# EUROPEAN PATENT SPECIFICATION

(11) **EP 4 050 101 B1**
(45) Date of publication and mention of the grant of the patent: **26.06.2024**
(21) Application number: 21739220.8
(22) Date of filing: 22.04.2021
(51) Int. Cl.: C12N 9/10, C12N 15/77, C12N 1/20, C12P 19/32

(54) **NOVEL PHYTOENE SYNTHASE VARIANT AND METHOD FOR PRODUCING XMP OR GMP USING SAME**
NEUARTIGE PHYTOENSYNTHASEVARIANTE UND VERFAHREN ZUR HERSTELLUNG VON XMP ODER GMP UNTER VERWENDUNG DERSELBEN
NOUVEAU VARIANT DE PHYTOÈNE SYNTHASE ET PROCÉDÉ DE PRODUCTION DE XMP OU GMP L'UTILISANT

(30) Priority: 15.01.2021 KR 20210006131
(43) Date of publication of application: 31.08.2022
(73) Proprietor: CJ CheilJedang Corporation, Seoul 04560 (KR)
(72) Inventor: BAE, Jee Yeon, Seoul 04560 (KR); SIM, Se Hoon, Seoul 04560 (KR); LEE, Ji Hyun, Seoul 04560 (KR); LEE, Ji Hye, Seoul 04560 (KR); PARK, Goun, Seoul 04560 (KR); KIM, Hyo Jin, Seoul 04560 (KR); SEO, Chang Il, Seoul 04560 (KR)
(74) Representative: Mewburn Ellis LLP
(86) International application number: PCT/KR2021/005074
(87) International publication number: WO 2022/154189

(56) References cited:
- EP-A1- 3 498 836
- JP-A- 2019 165 635
- KR-B1- 101 950 141
- KR-B1- 102 185 850
- US-A1- 2015 315 551
- US-A1- 2020 392 478
- DATABASE UNIPROT [Online] 25 October 2017 (2017-10-25), Yang J. ET AL: "Phytoene synthase", XP055929858, retrieved from EBI Database accession no. A0A241TXJ1
- SANCHEZ S ET AL: "Metabolic regulation and overproduction of primary metabolites", MICROBIAL BIOTECHNOLOGY, UNIVERSITY OF SHEFFIELD * BIOMEDICAL INFORMATION SERVICE (SUBIS), UNITED KINGDOM , vol. 1, no. 4 1 July 2008 (2008-07-01), pages 283-319, XP002767827, ISSN: 0964-7562, DOI: 10.1111/J.1751-7915.2007.00015.X Retrieved from the Internet: URL:http://onlinelibrary.wiley.com/doi/10. 1111/j.1751-7915.2007.00015.x/pdf [retrieved on 2008-02-01]
- DATABASE Protein 23 July 2017 (2017-07-23), Anonymous: "phytoene/squalene synthase family protein [Corynebacterium stationis]", XP055828144, retrieved from NCBI Database accession no. WP_075723423

## Description

### [Technical Field]

The present disclosure relates to a novel phytoene synthase variant, a *Corynebacterium stationis* strain comprising the variant, and a method for producing XMP or GMP using the strain.

### [Background Art]

Various studies are being conducted to develop highly efficient microorganisms and fermentation process technologies for the production of XMP, GMP, and other useful substances. For example, a target substance-specific approach is mainly used in which the expression of a gene encoding an enzyme involved in XMP or GMP biosynthesis is increased, or in which genes unnecessary for the biosynthesis are removed (EP 3722430A1, US 2020-0347346 A1).

However, it is still necessary to conduct studies to effectively increase the XMP and GMP producing ability as the demand for XMP and GMP increases.

### [Disclosure]

### [Technical Problem]

The present inventors have developed a novel phytoene synthase variant, a *Corynebacterium stationis* strain comprising the variant, and a method for producing XMP or GMP using the strain, thereby completing the present disclosure.

### [Technical Solution]

An object of the present disclosure is to provide a phytoene synthase variant consisting of an amino acid sequence represented by SEQ ID NO: 1, in which alanine, which is an amino acid corresponding to position 133 of an amino acid sequence of SEQ ID NO: 3, is substituted with valine.

Another object of the present disclosure is to provide a polynucleotide encoding the variant of the present disclosure.

Still another object of the present disclosure is to provide a *Corynebacterium stationis* strain that comprises the variant of the present disclosure or a polynucleotide encoding the variant and has XMP or GMP producing ability.

Still another object of the present disclosure is to provide a method for producing XMP or GMP, which comprises culturing a *Corynebacterium stationis* strain that comprises a variant of the present disclosure or a polynucleotide encoding the variant and has XMP or GMP producing ability in a medium.

The invention is set out in the appended set of claims.

### [Advantageous Effects]

In the case of culturing a *Corynebacterium stationis* strain comprising the phytoene synthase variant of the present disclosure, it is possible to produce XMP or GMP at a higher yield as compared to the case of existing microorganisms having unmodified polypeptides.

### [Description of Drawings]

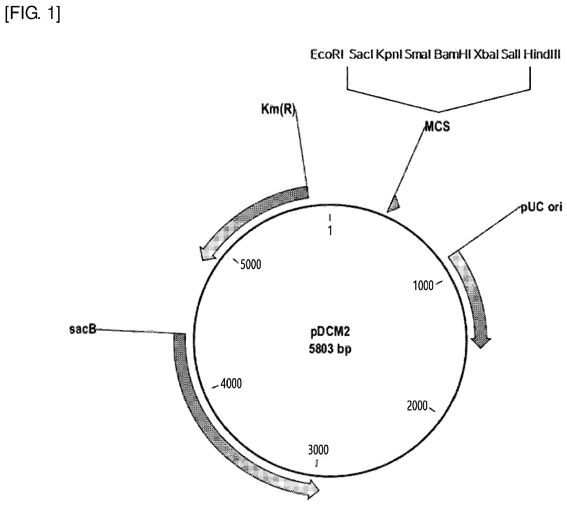

FIG. 1 is a schematic diagram of pDCM2 plasmid.

### [Best Mode]

The present disclosure will be described in detail as follows. Meanwhile, each of the descriptions and embodiments disclosed in the present disclosure may be applied to other descriptions and embodiments. In other words, all combinations of various elements disclosed in the present disclosure belong to the scope of the present disclosure. In addition, it cannot be considered that the scope of the present disclosure is limited by the specific description described below. In addition, throughout the present specification, a number of papers and patent documents are referenced and citations thereof indicated.

The present invention provides a phytoene synthase variant consisting of an amino acid sequence represented by SEQ ID NO: 1, in which alanine, which is an amino acid corresponding to position 133 of an amino acid sequence of SEQ ID NO: 3, is substituted with valine.

A variant of the present disclosure consists of the amino acid sequence represented by SEQ ID NO: 1.

In the variant of the present disclosure, the amino acid corresponding to position 133 based on the amino acid sequence of SEQ ID NO: 3 in the amino acid sequence represented by SEQ ID NO: 1 is valine.

For the purposes of the present disclosure, the variant is a polypeptide consisting of an amino acid sequence represented by SEQ ID NO: 1, in which alanine, which is an amino acid corresponding to position 133 of the amino acid sequence of SEQ ID NO: 3, is substituted with valine.

In the present disclosure, the term "homology" or "identity" means the degree of similarity between two given amino acid sequences or base sequences and may be expressed as a percentage. The terms homology and identity may often be used interchangeably.

The sequence homology or identity of a conserved polynucleotide or polypeptide is determined by standard alignment algorithms, and the default gap penalty established by the program used may be used together. Substantially, homologous or identical sequences are generally capable of being hybridized with the entirety or a part of the sequence in moderately or highly stringent conditions. It is apparent that hybridization also includes hybridization of a polynucleotide with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al., (1988) Proc. Natl. Acad. Sci. USA 85:2444. Alternatively, the homology, similarity, or identity may be determined using Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego, 1994; and CARILLO et al. (1988) SIAM J Applied Math 48:1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al., (1986) Nucl. Acids Res. 14:6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

As an example of the present disclosure, the variant of the present disclosure may exhibit phytoene synthase activity. The variant of the present disclosure may exhibit activity so as to have increased XMP or GMP producing ability as compared to a wild-type polypeptide exhibiting phytoene synthase activity.

In the present disclosure, the term "phytoene synthase" refers to a polypeptide involved in the biosynthesis of carotenoids. Specifically, the phytoene synthase of the present disclosure may be used interchangeably with CrtB2, or PSY In the present disclosure, the sequence of the phytoene synthase may be obtained from GenBank of the NCBI, a known database. Specifically, the phytoene synthase may be a polypeptide exhibiting phytoene synthase activity encoded by *crtB2,* but is not limited thereto.

In the present disclosure, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, "corresponding region" generally refers to a similar or corresponding position in a related protein or a reference protein.

For example, an arbitrary amino acid sequence is aligned with SEQ ID NO: 3, and based on this, each amino acid residue of the amino acid sequence may be numbered with reference to the amino acid residue of SEQ ID NO: 3 and the numerical position of the corresponding amino acid residue. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48:443-453), the Needleman program of the EMBOSS package (EMBOSS: The European Molecular Biology Open Software Suite, Rice et al., 2000, Trends Genet. 16:276-277), and the like may be used, but the program and algorithm are not limited thereto, and a sequence alignment program, a pairwise sequence comparison algorithm, and the like known in the art may be appropriately used.

Another aspect of the present disclosure is to provide a polynucleotide encoding the variant of the present disclosure. The invention is set out in the appended set of claims.

In the present disclosure, the term "polynucleotide" is a DNA or RNA strand having a certain length or more as a polymer of nucleotides in which nucleotide monomers are connected in a long chain by covalent bonds, and more specifically means a polynucleotide fragment encoding the variant.

The polynucleotide encoding the variant of the present disclosure may comprise a base sequence encoding the amino acid sequence represented by SEQ ID NO: 1. As an example of the present disclosure, the polynucleotide of the present disclosure may have or comprise the sequence of SEQ ID NO: 2. The polynucleotide of the present disclosure may consist of or consist essentially of the sequence of SEQ ID NO: 2.

In the polynucleotide of the present disclosure, various modifications may be made in the coding region as long as the amino acid sequence of the variant of the present disclosure is not changed in consideration of codon degeneracy or codons preferred in organisms that are intended to express the variant of the present disclosure. Specifically, the polynucleotide of the present disclosure has or comprises a base sequence having 70% or more, 75% or more, 80% or more, 85% or more, 90% or more, 95% or more, 96% or more, 97% or more, 98% or more, but less than 100% homology or identity to the sequence of SEQ ID NO: 2 or may consist of or consist essentially of a base sequence having 70% or more, 75% or more, 80% or more, 85% or more, 90% or more, 95% or more, 96% or more, 97% or more, 98% or more, but less than 100% homology or identity to the sequence of SEQ ID NO: 2, but is not limited thereto. Here, in the sequence having homology or identity, the codon encoding the amino acid corresponding to position 133 of SEQ ID NO: 1 is one of the codons encoding valine.

The polynucleotide of the present disclosure may comprise a probe that can be prepared from a known gene sequence, for example, a sequence without limitation as long as it is a sequence that can hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, namely polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed one time, specifically two to three times at a salt concentration and temperature equivalent to 60°C, 1X SSC, 0.1% SDS, specifically 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tₘ value of 55°C and the above-described conditions. The Tₘ value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (for example, J. Sambrook et al., Molecular Cloning, A Laboratory Manual, 2nd Edition, Cold Spring Harbor Laboratory press, Cold Spring Harbor, New York, 1989).

Another aspect of the present disclosure is to provide a vector comprising the polynucleotide of the present disclosure. The vector may be an expression vector for expressing the polynucleotide in a host cell, but is not limited thereto.

The vector of the present disclosure may include a DNA construct comprising a polynucleotide sequence encoding a polypeptide of interest operably linked to a suitable expression control region (or expression control sequence) so that the polypeptide of interest may be expressed in a suitable host. The expression control region may contain a promoter capable of initiating transcription, any operator sequence for controlling the transcription, a sequence encoding a suitable mRNA ribosome binding site, and a sequence controlling termination of transcription and translation. The vector may be transformed into a suitable host cell and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, and the like may be used as a phage vector or a cosmid vector, and pDZ system, pBR system, pUC system, pBluescript II system, pGEM system, pTZ system, pCL system, pET system, and the like may be used as a plasmid vector. Specifically, pDZ, pDC, pDCM2, pACYC177, pACYC184, pCL, pECCG117, pUC19, pBR322, pMW118, and pCC1BAC vectors and the like may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further contain a selection marker for the confirmation of chromosome insertion. The selection marker is for selecting the cells transformed with vectors, that is, for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells can be selected.

In the present disclosure, the term "transformation" means that a vector comprising a polynucleotide encoding a target polypeptide is introduced into a host cell or a microorganism so that the polypeptide encoded by the polynucleotide can be expressed in the host cell. The transformed polynucleotide may be located by being inserted into the chromosome of the host cell or located outside the chromosome as long as it can be expressed in the host cell. The polynucleotide comprises DNA and/or RNA encoding a polypeptide of interest. The polynucleotide may be introduced in any form as long as it can be introduced into a host cell and expressed. For example, the polynucleotide may be introduced into a host cell in the form of an expression cassette, which is a gene construct containing all elements required for self-expression. The expression cassette may usually contain a promoter operably linked to the polynucleotide, a transcription termination signal, a ribosome binding site, and a translation termination signal. The expression cassette may be in the form of an expression vector capable of self-replicating. The polynucleotide may be introduced into a host cell in its own form and operably linked to a sequence required for expression in the host cell, but is not limited thereto.

In the above, the term "operably linked" means that the polynucleotide sequence is functionally linked to a promoter sequence that initiates and mediates transcription of the polynucleotide encoding the variant of interest of the present disclosure.

Still another aspect of the present disclosure is to provide a *Corynebacterium stationis* strain that comprises the variant of the present disclosure or the polynucleotide of the present disclosure. The invention is set out in the appended set of claims.

The strain of the present disclosure may comprise a modified polypeptide of the present disclosure, a polynucleotide encoding the polypeptide, or a vector comprising the polynucleotide of the present disclosure.

In the present disclosure, the "strain (or microorganism)" includes all wild-type microorganisms or naturally or artificially genetically modified microorganisms, and it may be a microorganism in which a specific mechanism is weakened or strengthened due to a insertion of an external gene or an activity enhancement or inactivation of an endogenous gene, and it may be a microorganism comprising genetic modification for production of a polypeptide, protein, or product of interest.

The strain of the present disclosure may be a strain comprising any one or more of the variant of the present disclosure, the polynucleotide of the present disclosure, or a vector comprising the polynucleotide of the present disclosure; a strain modified to express the variant of the present disclosure or the polynucleotide of the present disclosure; a strain (for example, a recombinant strain) expressing the variant of the present disclosure or the polynucleotide of the present disclosure; or a strain (for example, a recombinant strain) exhibiting the activity of the variant of the present disclosure, but is not limited thereto.

The strain of the present disclosure may be a strain having 5'-xanthosine monophosphate (XMP) or 5'-guanosine monophosphate (GMP) producing ability.

The strain of the present disclosure may be a microorganism naturally having phytoene synthase or XMP or GMP producing ability or a microorganism in which the variant of the present disclosure or a polynucleotide encoding the variant (or a vector comprising the polynucleotide) is introduced into a parent strain that does not have phytoene synthase or XMP or GMP producing ability and/or in which XMP or GMP producing ability is conferred on the parent strain, but is not limited thereto.

For example, the strain of the present disclosure is a cell or microorganism that is transformed with a vector comprising the polynucleotide of the present disclosure or a polynucleotide encoding the variant of the present disclosure and expresses the variant of the present disclosure. For the purposes of the present disclosure, the strain of the present disclosure may include all microorganisms that comprise the variant of the present disclosure and can produce XMP or GMP. For example, the strain of the present disclosure may be a recombinant strain in which a polynucleotide encoding the variant of the present disclosure is introduced into a natural wild-type microorganism or a microorganism producing XMP or GMP to thus express a phytoene synthase variant and having increased XMP or GMP producing ability. The recombinant strain having increased XMP or GMP producing ability may be a microorganism having increased XMP or GMP producing ability as compared to a natural wild-type microorganism or a phytoene synthase unmodified microorganism (namely, a microorganism expressing wild-type phytoene synthase (SEQ ID NO: 3) or a microorganism that does not express modified (SEQ ID NO: 1) protein), but is not limited thereto. The strain having increased XMP or GMP producing ability of the present disclosure is a microorganism having increased XMP or GMP producing ability as compared to *Corynebacterium stationis* which comprises a polypeptide of SEQ ID NO: 3 or a polynucleotide encoding the polypeptide.

As an example, the phytoene synthase unmodified microorganism that is a target strain for comparison of the increase in XMP or GMP producing ability may be an ATCC6872 or CJX1664 strain (KCCM12285P, KR 10-1950141 B1), but is not limited thereto.

For example, the recombinant strain having increased producing ability may have XMP or GMP producing ability increased by about 1% or more, specifically about 1% or more, about 2.5% or more, about 5% or more, about 6% or more, about 7% or more, about 8% or more, about 9% or more, about 10% or more, about 10.5% or more, about 11% or more, about 11.5% or more, about 12% or more, about 12.5% or more, about 13% or more, about 13.5% or more, about 14% or more, or about 14.5% or more (the upper limit is not particularly limited and may be, for example, about 200% or less, about 150% or less, about 100% or less, about 50% or less, about 45% or less, about 40% or less, about 35% or less, about 30% or less, about 25% or less, about 20% or less, or about 15% or less) as compared to the XMP or GMP producing ability of the parent strain before being varied or an unmodified microorganism, but the increased amount is not limited thereto as long as the producing ability has an increased amount of a + value as compared to the producing ability of the parent strain before being varied or an unmodified microorganism. In another example, the recombinant strain having increased producing ability may have XMP or GMP producing ability increased by about 1.1 times or more, about 1.12 times or more, about 1.13 times or more, or about 1.14 times or more (the upper limit is not particularly limited and may be, for example, about 10 times or less, about 5 times or less, about 3 times or less, or about 2 times or less) as compared to the XMP or GMP producing ability of the parent strain before being varied or an unmodified microorganism, but the rate of increase is not limited thereto. The term "about" is a range including all of ±0.5, ±0.4, ±0.3, ±0.2, ±0.1, and the like, includes all values in a range equal to or similar to the value following the term "about", but is not limited thereto.

In the present disclosure, the "unmodified microorganism" does not exclude strains comprising mutation that may occur naturally in microorganisms, and may be a wild-type strain or a natural strain itself or may be a strain before the trait is changed by genetic variation due to natural or artificial factors. For example, the unmodified microorganism may be a strain into which the phytoene synthase variant described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

In another example of the present disclosure, the microorganism of the present disclosure is *Corynebacterium stationis.*

In the microorganism of the present disclosure, partial or entire modification of a polynucleotide may be induced by (a) homologous recombination using a vector for chromosome insertion in the microorganism or genome editing using engineered nuclease (*e.g.*, CRISPR-Cas9) and/or (b) treatment with light such as ultraviolet rays and radiation and/or chemicals, but is not limited thereto. A method for modifying a part or the entirety of the gene may include a method using DNA recombination technology. For example, by introducing a nucleotide sequence or vector containing a nucleotide sequence homologous to the gene of interest into the microorganism to cause homologous recombination, a part or the entirety of the gene may be deleted. The introduced nucleotide sequence or vector may contain a dominant selection marker, but is not limited thereto.

In the microorganism of the present disclosure, the variant, polynucleotide, XMP and GMP, and the like are as described in the other aspects.

Still another aspect of the present disclosure provides a method for producing XMP or GMP, which includes culturing a *Corynebacterium stationis* strain comprising the variant of the present disclosure or the polynucleotide of the present disclosure in a medium. The invention is set out in the appended set of claims.

The method for producing XMP or GMP of the present disclosure may include culturing a *Corynebacterium stationis* strain comprising the variant of the present disclosure or the polynucleotide of the present disclosure or the vector of the present disclosure in a medium.

In the present disclosure, the term "culture" means growing the *Corynebacterium stationis* strain of the present disclosure under appropriately controlled environmental conditions. The culture process of the present disclosure may be performed according to suitable medium and culture conditions known in the art. Such a culture process may be easily adjusted and used by those skilled in the art according to the selected strain. Specifically, the culture may be a batch type, continuous type, and/or fed-batch type, but is not limited thereto.

In the present disclosure, the term "medium" means a mixed substance containing nutrients required to culture the *Corynebacterium stationis* strain of the present disclosure as a main component, and the medium supplies nutrients, growth factors, and the like including water that are indispensable for survival and development. Specifically, as the medium and other culture conditions used for culture of the *Corynebacterium stationis* strain of the present disclosure, any one may be used without particular limitation as long as it is a medium used for common culture of microorganisms. The *Corynebacterium stationis* strain of the present disclosure may be cultured in a common medium containing proper carbon sources, nitrogen sources, phosphorus sources, inorganic compounds, amino acids and/or vitamins, and the like while controlling the temperature, pH, and the like under aerobic conditions.

Specifically, the culture medium for the strain of the genus *Corynebacterium* may be found in the document "Manual of Methods for General Bacteriology" by the American Society for Bacteriology (Washington, D.C., USA, 1981).

In the present disclosure, the carbon sources include carbohydrates such as glucose, saccharose, lactose, fructose, sucrose, and maltose; sugar alcohols such as mannitol and sorbitol, organic acids such as pyruvic acid, lactic acid, and citric acid; amino acids such as glutamic acid, methionine, and lysine; and the like. Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (namely, molasses converted to reducing sugar) may be used, and appropriate amounts of other carbon sources may be used in various manners without limitation. These carbon sources may be used singly or in a combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in a combination of two or more, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, and the like may be used. In addition to these, amino acids, vitamins and/or suitable precursors, and the like may be contained. These components or precursors may be added to the medium batchwise or continuously, but the manner of addition is not limited thereto.

During the culture of the *Corynebacterium stationis* strain of the present disclosure, the pH of the medium may be adjusted by adding compounds such as ammonium hydroxide, potassium hydroxide, ammonia, phosphoric acid, and sulfuric acid to the medium in a proper manner. During the culture, foaming may be suppressed by using an antifoaming agent such as fatty acid polyglycol ester. Oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but the method for maintaining the state is not limited thereto.

In the culture of the present disclosure, the culture temperature may be maintained at 20°C to 45°C, specifically 25°C to 40°C, and the strain may be cultured for about 10 to 160 hours, but the culture conditions are not limited thereto.

XMP or GMP produced through the culture of the present disclosure may be secreted into the medium or may remain in the cells.

The method for producing XMP or GMP of the present disclosure may further comprise a step of preparing the *Corynebacterium stationis* strain of the present disclosure, a step of preparing a medium for culture of the strain, or a combination of these (in any order), for example, prior to the culture step.

The method for producing XMP or GMP of the present disclosure may further include a step of recovering XMP or GMP from the medium according to the culture (the medium subjected to the culture) or from the *Corynebacterium stationis* strain. The recovery step may be further included after the culture step.

The recovery may be to collect the XMP or GMP of interest by way of a suitable method known in the art according to the method for culturing the microorganism of the present disclosure, for example, a batch, continuous, or fed-batch culture method. For example, centrifugation, filtration, treatment with a crystallized protein precipitant (salting out), extraction, ultrasonic disintegration, ultrafiltration, dialysis, various forms of chromatography such as molecular-sieve chromatography (gel filtration), adsorption chromatography, ion-exchange chromatography, and affinity chromatography, HPLC, or a combination thereof may be used. The XMP or GMP of interest may be recovered from the medium or microorganism by way of a suitable method known in the art.

The method for producing XMP or GMP of the present disclosure may further include a purification step. The purification may be performed by way of a suitable method known in the art. In an example, when the method for producing XMP or GMP of the present disclosure includes both the recovery step and the purification step, the recovery step and the purification step may be performed continuously or discontinuously regardless of the order, or may be performed simultaneously or by being combined into one step, but the manner to perform the steps is not limited thereto.

Specifically, the method for producing GMP of the present disclosure may further include a step of converting the XMP to GMP. In the method for producing GMP of the present disclosure, the conversion step may be further included after the culture step or the recovery step. The conversion step may be performed by way of a suitable method known in the art. For example, the conversion may be performed using a coryneform microorganism, *E. coli,* or 5'-xanthylate aminase (KR 10-0655902 B1), but the manner to perform the conversion is not limited thereto.

In the method of the present disclosure, the variant, polynucleotide, vector, strain, XMP and GMP, and the like are as described in the other aspects.

Still another aspect of the present disclosure is to provide a composition for XMP or GMP production, which comprises a *Corynebacterium stationis* strain comprising the variant of the present disclosure, a polynucleotide encoding the variant, a vector comprising the polynucleotide, or the polynucleotide of the present disclosure; a medium in which this *Corynebacterium stationis* strain has been cultured; or a combination of two or more of these.

The composition of the present disclosure may further comprise arbitrary suitable excipients to be commonly used in compositions for XMP or GMP production. Such excipients may be, for example, a preservative, a wetting agent, a dispersing agent, a suspending agent, a buffering agent, a stabilizer, or an isotonic agent, but are not limited thereto.

In the composition of the present disclosure, the variant, polynucleotide, vector, strain, medium, XMP and GMP, and the like are as described in the other aspects.

### [Mode for Invention]

Hereinafter, the present disclosure will be described in more detail by way of Examples. However, the following Examples are only preferred embodiments for illustrating the present disclosure, and thus are not intended to limit the scope of the present disclosure thereto. Meanwhile, technical matters not described in the present specification can be sufficiently understood and easily implemented by those skilled in the technical field of the present disclosure or similar technical fields.

### Example 1: Construction of plasmid

A plasmid (pDCM2, FIG. 1, SEQ ID NO: 11) for the insertion and replacement of genes in the *Corynebacterium* chromosome was designed and synthesized using the Gene-synthesis service of BIONICS Co., Ltd. The plasmid was designed to contain restriction enzyme sites to be easily used for cloning by referring to a commonly known paper on the sacB system (Gene, 145 (1994) 69-73). The pDCM2 plasmid thus synthesized has the following properties:
1) The pDCM2 plasmid has a replication origin that works only in *E. coli,* and self-replication is thus possible in *E. coli* but not in *Corynebacterium.*
2) The pDCM2 plasmid has a kanamycin resistance gene as a selection marker.
3) The pDCM2 plasmid has a Levan sucrose gene (sacB) as a secondary positive-selection marker.
4) No gene information derived from the pDCM2 plasmid remains in the finally constructed strain.

### Example 2. Construction of vector for phytoene synthase variant expression in microorganism

In order to explore the effect of a variant (A133V; SEQ ID NO: 1), in which alanine at position 133 of a phytoene synthase amino acid sequence (SEQ ID NO: 3) was substituted with valine, on XMP production, a vector for the construction of a strain expressing the variant was constructed as follows.

PCR was performed using gDNA (genomic DNA) of wild-type *Corynebacterium stationis* ATCC6872 as a template and a pair of primers having sequences of SEQ ID NOs: 5 and 6 and a pair of primers having sequences of SEQ ID NOs: 7 and 8. Overlapping PCR was performed again using the mixture of two fragments obtained above as a template and a pair of primers having sequences of SEQ ID NO: 5 and SEQ ID NO: 8 to obtain a fragment. The PCR was performed as follows: denaturation at 94°C for 5 minutes; 30 cycles of 94°C for 30 seconds, 55°C for 30 seconds, and 72°C for 1 minute 30 seconds; and 72°C for 5 minutes. The pDCM2 vector was treated with smal, and the PCR product obtained above was fusion cloned thereinto. Fusion cloning was performed using an In-Fusion^{®} HD cloning kit (Clontech). The obtained plasmid was named pDCM2-crtB2(A133V).

### Example 3: Evaluation on XMP producing ability of microorganism expressing phytoene synthase variant

### 3-1. Construction of strain for expressing phytoene synthase variant

The vector constructed in Example 2 was transformed into *Corynebacterium stationis* CJX1664 strain (KCCM12285P, KR 10-1950141 B1).

The strain into which the variant was introduced was selected from the strains in which homologous recombination took place using SEQ ID NOs: 9 and 10. The selected strain was named CJX1664_crtB2_A133V.

### 3-2. Comparison of XMP producing ability of strains expressing phytoene synthase variant

The XMP producing ability was analyzed through flask fermentation titer evaluation of each strain constructed in Example 3-1 and a control parent strain.

First, each strain was inoculated into a test tube with a diameter of 18 mm containing 2 mL of seed medium and shake-cultured for 24 hours at 30°C to be used as a seed culture solution. Into a 250 mL corner-baffle flask containing 32 mL of the following production medium (24 mL of main medium + 8 mL of separate sterilization medium), 0.7 mL of the seed culture solution was inoculated and shake-cultured at 170 rpm for 75 hours at 30°C. After completion of the culture, the XMP producing ability was measured by HPLC. The XMP concentration in the culture solution for each of the tested strains and the rate of increase in the XMP concentration are as presented in Table 1 below.

### <Seed medium (pH 7.5)>

Glucose 1%, peptone 1%, beef extract 1%, yeast extract 1%, sodium chloride 0.25%, adenine 100 mg/L, and guanine 100 mg/L (based on 1 liter of distilled water)

### <XMP flask production medium (main medium)>

Glucose 40 g/L, magnesium sulfate 10 g/L, calcium chloride 100 mg/L, iron sulfate 20 mg/L, manganese sulfate 10 mg/L, zinc sulfate 10 mg/L, copper sulfate 0.8 mg/L, histidine 20 mg/L, cystine 15 mg/L, beta-alanine 15 mg/L, biotin 100 µg/L, thiamine 5 mg/L, adenine 50 mg/L, guanine 25 mg/L, niacin 15 mg/L, and pH 7.0

### <XMP flask production medium (separate sterilization medium)>

Monopotassium phosphate 18 g/L, dipotassium phosphate 42 g/L, urea 7 g/L, and ammonium sulfate 5 g/L

The experiment was repeated 3 times, and the average values of the analysis results thereof are presented in Table 1 below.

**[Table 1]**

| Comparison of XMP producing ability | | | |
|---|---|---|---|
| Strain | | XMP concentration (g/L) | Rate of increase in XMP concentration (%) |
| | CJX1664 | 4.72 | - |
| | CJX1664_crtB2_A133V | 5.40 | 14.4 |

As presented in Table 1, the CJX1664_crtB2_A133V strain exhibited XMP producing ability increased by 14.4% as compared to that of the control group.

CJX1664_crtB2_A133V was named CN02-2071, deposited with the Korea Culture Center of Microorganisms, a depository institution under the Budapest Treaty, on December 2, 2020, and given the accession number KCCM12877P.

### [Accession Number]

Depository institution: Korea Culture Center of Microorganisms
Accession number: KCCM12877P
Accession date: 20201202

## Claims

1. A phytoene synthase variant consisting of an amino acid sequence represented by SEQ ID NO: 1, wherein alanine, which is an amino acid corresponding to position 133 of SEQ ID NO: 3, is substituted with valine.

2. A polynucleotide encoding the variant according to claim 1.

3. A *Corynebacterium stationis* strain comprising the variant according to claim 1 or a polynucleotide encoding the variant, which has increased 5'-xanthosine monophosphate (XMP) producing ability as compared to *Corynebacterium stationis* comprising a polypeptide of SEQ ID NO: 3 or a polynucleotide encoding the polypeptide.

4. The strain according to claim 3, wherein the 5'-xanthosine monophosphate (XMP) is converted to 5'-guanosine monophosphate (GMP).

5. A method for producing 5'-xanthosine monophosphate (XMP), the method comprising culturing a *Corynebacterium stationis* strain comprising the variant according to claim 1 or a polynucleotide encoding the variant in a medium,
wherein the strain has increased 5'-xanthosine monophosphate (XMP) producing ability as compared to *Corynebacterium stationis* comprising a polypeptide of SEQ ID NO: 3 or a polynucleotide encoding the polypeptide.

6. A method for producing 5'-guanosine monophosphate (GMP), the method comprising (a) producing 5'-xanthosine monophosphate (XMP) according to the method of claim 5; and (b) converting the 5'-xanthosine monophosphate (XMP) produced in step (a) to 5'-guanosine monophosphate (GMP).

## Patentansprüche

1. Phytoen-Synthasevariante, bestehend aus einer Aminosäuresequenz, dargestellt durch SEQ ID NO: 1, wobei Alanin, das eine Aminosäure ist, die Position 133 von SEQ ID NO: 3 entspricht, durch Valin substituiert ist.

2. Polynucleotid, das für eine Variante nach Anspruch 1 kodiert.

3. Corynebacterium-stationis-Stamm, umfassend eine Variante nach Anspruch 1 oder ein Polynucleotid, das für die Variante kodiert, der verglichen mit Corynebacterium stationis, umfassend ein Polypeptid von SEQ ID NO: 3 oder ein Polynucleotid, das für das Polypeptid kodiert, eine verstärkte Fähigkeit zur Produktion von 5'-Xanthosinmonophosphat (XMP) aufweist.

4. Stamm nach Anspruch 3, wobei das 5'-Xanthosinmonophosphat (XMP) in 5'-Guanosinmonophosphat (GMP) umgewandelt wird.

5. Verfahren zur Herstellung von 5'-Xanthosinmonophosphat (XMP), wobei das Verfahren das Kultivieren eines Corynebacterium-stationis-Stamms, umfassend eine Variante nach Anspruch 1 oder ein Polynucleotid, das für die Variante kodiert, in einem Medium umfasst,
wobei der Stamm verglichen mit Corynebacterium stationis, umfassend ein Polypeptid von SEQ ID NO: 3 oder ein Polynucleotid, das für das Polypeptid kodiert, eine verstärkte Fähigkeit zur Produktion von 5`-Xanthosinmonophosphat (XMP) aufweist.

6. Verfahren zur Herstellung von 5'-Guanosinmonophosphat (GMP), wobei das Verfahren (a) das Herstellen von 5'-Xanthosinmonophosphat (XMP) nach einem Verfahren aus Anspruch 5; und (b) das Umwandeln des in Schritt (a) hergestellten 5'-Xanthosinmonophosphats (XMP) in 5'-Guanosinmonophosphat (GMP) umfasst.

## Revendications

1. Variant de phytoène synthase composé d'une séquence d'acides aminés représentée par SEQ ID NO : 1, dans lequel l'alanine, qui est un acide aminé correspondant à la position 133 de SEQ ID NO : 3, est substituée par de la valine.

2. Polynucléotide codant pour le variant selon la revendication 1.

3. Souche de Corynebacterium stationis comprenant le variant selon la revendication 1 ou un polynucléotide codant pour le variant, qui présente une capacité de production de 5'-xanthosine monophosphate (XMP) accrue par rapport au Corynebacterium stationis comprenant un polypeptide de SEQ ID NO : 3 ou un polynucléotide codant pour le polypeptide.

4. Souche selon la revendication 3, dans laquelle le 5'-xanthosine monophosphate (XMP) est converti en 5'-guanosine monophosphate (GMP).

5. Procédé de production de 5'-xanthosine monophosphate (XMP), le procédé comprenant une mise en culture d'une souche de Corynebacterium stationis comprenant le variant selon la revendication 1 ou un polynucléotide codant pour le variant dans un milieu.
dans lequel la souche présente une capacité de production de 5'-xanthosine monophosphate (XMP) accrue par rapport au Corynebacterium stationis comprenant un polypeptide de SEQ ID NO : 3 ou un polynucléotide codant pour le polypeptide.

6. Procédé de production de 5'-guanosine monophosphate (GMP), le procédé comprenant (a) une production de 5'-xanthosine monophosphate (XMP) selon le procédé de la revendication 5 ; et (b) une conversion de 5'-xanthosine monophosphate (XMP) produit à l'étape (a) en 5'-guanosine monophosphate (GMP).
